# EUROPEAN PATENT SPECIFICATION

(11) **EP 2 099 466 B1**
(45) Date of publication and mention of the grant of the patent: **03.06.2015**
(21) Application number: 07864012.5
(22) Date of filing: 07.11.2007
(51) Int. Cl.: A61K 38/00, A61K 38/44, A61K 38/47, A61K 47/10, A61P 31/04

(54) **USE OF HYDROLYTIC AND OXIDATIVE ENZYMES TO DISSOLVE BIOFILM IN EARS**
VERWENDUNG VON HYDROLYTISCHEN UND OXIDATIVEN ENZYMEN ZUR AUFLÖSUNG VON BIOFIMEN IN OHREN
UTILISATION D'ENZYMES HYDROLYTIQUES ET OXYDATIVES POUR DISSOUDRE UN BIOFILM DANS LES OREILLES

(30) Priority: 01.12.2006 US 868131 P; 15.12.2006 US 870328 P
(43) Date of publication of application: 16.09.2009
(73) Proprietor: Laclede, Inc., Rancho Dominguez, CA 90220 (US)
(72) Inventor: PELLICO, Michael, Rancho Dominquez Drive, CA 90220 (US)
(74) Representative: Winter, Brandl, Fürniss, Hübner, Röss, Kaiser, Polte - Partnerschaft mbB
(86) International application number: PCT/US2007/083877
(87) International publication number: WO 2008/070387

(56) References cited:
- WO-A1-01/53010
- US-A1- 2002 037 260
- US-A1- 2005 158 253
- US-B1- 6 214 339
- JOHANSEN C ET AL: "Enzymatic removal and disinfection of bacterial biofilms.", APPLIED AND ENVIRONMENTAL MICROBIOLOGY SEP 1997 LNKD- PUBMED:9293025, vol. 63, no. 9, September 1997 (1997-09), pages 3724-3728, XP002670470, ISSN: 0099-2240

## Description

### FIELD OF THE INVENTION

This invention relates to ear cleaning compositions where the cleaning composition contains biofilm dissolving enzymes, in particular hydrolytic enzymes and oxidative enzymes.

### BACKGROUND OF THE INVENTION

Ear cleaning compositions are formulated to contain ingredients for the prevention and treatment of ear infections.

Otitis media and otitis externa are particularly common ear infections. Although common, these ear infections, including otitis media and otitis externa, can have severe consequences. Three out of four children experience otitis media by the time that they are three years old.

There are two main types of otitis media. The first type is called acute otitis media (AOM). Parts of the ear are infected and swollen. Fluid and mucus are trapped inside the ear. AOM can be very painful.

The second type is called otitis media with effusion(fluid) or OME. This means that fluid and mucus stay trapped in the ear after the infection is thought to be over. OME often leads to new infections and can affect a child's hearing. Hearing loss, especially in children, can impair learning capacity and even delay speech development. Otitis media is also serious because the infection can spread to nearby structures in the head, especially the mastoid. Infections of the mastoid are often extremely difficult to treat, even with broad-spectrum antibiotics.

Otitis media or otitis externa usually happens when viruses, bacteria, or yeast/fungi find their way into the middle or external ear canal and cause an infection. Often after the acute infection has passed, the effusion remains and becomes chronic, lasting for weeks, months, or even years. This condition makes one subject to frequent recurrences of the acute infection.

The viruses that cause ear infections are thought to be the same ones that cause upper respiratory infections such as influenza or coryza (the common cold). One of the bacteria associated with ear infection is *Pseudomonas aeruginosa.* It is resistant to almost every possible antibiotic. The unfortunate tendency is for most bacteria to be killed off, leaving infection with the very resistant and practically immortal *P. aeruginosa.*

Current treatment for antibiotics is still with antibiotics, antiseptics, and fungicides. More often now, however, doctors are taking a wait and see attitude. Part of the reason that antibiotics have come into disfavor for treating ear infections is the fear that overuse of antibiotics will lead to a proliferation of antibiotic-resistant bacteria. It has been known for many years that bacteria have the ability to spread antibiotic resistance from one species to another through the action of plasmids known as resistance transfer factors (RTFs). Thus, if a relatively harmless commensal such as *Escherichia coli* becomes resistant to a particular antibiotic administered to a patient in response to an ear infection, the resistance to that antibiotic can be spread to other bacterial species such as the much more pathogenic *P*. *aeruginosa,* and once spread can be inherited in *P. aeruginosa.* Of particular significance is the generation of plasmids that carry multiple resistance genes. These mechanisms are described in A.A. Sayers and D.D. Whitt, "Bacterial Pathogenesis: A Molecular Approach" (ASM Press, Washington, DC 1994), pp. 107-109.

What has been shown repeatedly is that the resistance rate of common bacteria in countries that universally prescribe antibiotics for ear infections is much higher than in countries that do not routinely prescribe antibiotics. For example, researchers have found that 56% of children who had been prescribed antibiotics harbored multidrug-resistant bacteria in their noses, compared with 28% of the others.

The reason that antibiotics are frequently ineffective in this clinical situation is that recently it has been discovered that bacteria are living in a dormant state inside a slimy biofilm. The seemingly innocuous fluid behind the ear is actually a microbe-laden biofilm containing bacteria that become activated and grow rapidly under the right circumstances. This biofilm is also in the outer ear canal causing recurrent chronic otitis externa, resistant to most antibiotics. Otitis externa is typically associated with discomfort that is limited to the external auditory canal, with erythema (redness), and with swelling of the canal with variable discharge. Excessive moisture and trauma can predispose an individual to the occurrence of otitis externa. Otitis externa can be disabling enough to cause a significant fraction of patients to interrupt their daily activities for several days, typically requiring bed rest. If otitis externa is not optimally treated, especially in immunocompromised patients, the potentially life-threatening infection can spread to the surrounding tissues with extremely serious consequences. Immunocompromised patients include those patients with an immune system deficiency such as that occurring as a result of HIV infection, and those patients taking immunosuppressants such as tacrolimus to prevent transplant rejection. Otitis externa can occur in conjunction with otitis media; the latter can result from the spread of otitis externa. Like otitis media, otitis externa can spread into the mastoid and generate an extremely serious infection, one that can have a mortality rate exceeding 50%.

This revised understanding has come about because, previously, scientists studied bacteria in their free-floating form. Bacteria prefer the slimy, communal life because it protects them from toxins in the environment. Biofilm formation takes place in a step by step manner. First, inorganic or organic molecules are adsorbed to a surface. This creates a conditioning layer that increases the ability of bacteria to attach to a surface. Once a conditioning layer is formed, bacterial adhesion follows. Live or dead cells will attach to surfaces with similar propensity. Bacterial attachment is mediated by fimbriae, pili, and flagella, and by extracellular polysaccharides.

When first formed, the bond between the conditioning layer and the bacteria is not strong and can be easily removed. With time, however, these bonds are strengthened making removal difficult. Once embedded within a biofilm, bacterial cells have an opportunity to repair cellular damage and to metabolize nutrients within the biofilm. As the biofilm continues to grow, the extracellular polysaccharides provide more and more protection. A biofilm is mature within 24 hours. Biofilm development can occur within one hour. After an eight-hour period, more than 91% of the bacteria are strongly attached within the biofilm. Killing bacteria within a biofilm requires up to 1000 times more antibiotic than is required to kill free-floating bacteria. The film physically prevents the antibiotic from reaching the bacteria. In addition, most bacteria in the biofilm are dormant and antibiotics typically only kill bacteria that are actively dividing.

The eardrum is coated with a slimy reservoir of hibernating bacteria. These inactive bacteria do not cause symptoms of an active infection but eventually they slough off and become free-floating active bacteria and cause another infection. This is one of the significant factors behind the existence of recurrent infections in such patients. Data show that bacteria incorporated in biofilms are more resistant that single cells and this is believed to be caused by physical protection by the biofilm matrix or by altered physiology of bacterial cells in the biofilm.

Bacteria have a natural tendency to attach to surfaces and to initiate the formation of a biofilm. The biofilm matrix is a collection of microcolonies with water channels in between and an assortment of cells and extracellular polymers such as polysaccharides, glycoproteins, and proteins. The different types of bonds between the saccharides give rise to a large number of different classes of polysaccharides including levans, dextrans, cellulose, glycogen, and alginates. Bacteria have the capacity to attach to and to colonize the surface of most materials. Attachment often results in the production of extracellular polysaccharides and changes in cellular morphology and growth rates. Different genes are expressed in bacteria that are attached to surfaces as compared to planktonic bacteria. As a result, surface-attached bacteria display increased resistance to toxic chemicals and biocides. While biocides have proven effective in killing free-floating bacteria, they are not effective in destroying bacteria within a biofilm. It becomes imperative that the biofilm be destroyed before the biocides can become effective.

There are many methods known to remove biofilms. The methods that are used to remove biofilm include the use of hypochlorite, hydrogen peroxide, ozone, detergents, or acids, the application of heat, the use of mechanical activity, or the use of ultrasound. Combinations of these methods are also used.

Many of these methods, although effective, are not suitable for use on biofilms that form on the body or within the body. These methods are too harsh and disruptive of tissue for use in this context. A safe method is required to remove biofilms that form on the body or within the body.

Enzymes have been used to dissolve biofilms before, but not in the context of biofilms that form on the body or within the body. In laundry detergents, enzymes are used to remove deposits that may, in fact, be biofilms. Contact lens solutions use enzymes to remove the biofilm that can grow on a contact lens. In the dental field, dextranase and mutanase are used to remove plaque, a biofilm, from teeth.

Bactericidal activity as well as removal of the biofilm of *Pseudomonas aeruginosa* by the enzymatic combination of lactoperoxidase and glucose oxidase with polysaccharide-hydrolyzing enzymes are described in Johansen C et al., "Enzymatic removal and disinfection of bacterial biofilms", 1997, AEM, vol. 63, no.9., 3724-3728.

US2005/158253 A1 discloses the composition for degrading biofilms comprising an enzyme and an anchor molecule. The composition may further comprise hexose oxidase or lactoperoxidase.

US2002/037260 A1 discloses the composition for treating a biofilm structure comprising a first enzyme-anchor component for degrading the biofilm structure, and a second enzyme-anchor component acting directly bactericidaly. The second enzyme-anchor component comprises an enzyme selected from the group of: oxido-reductase, peroxidase, hexose oxidase, lactoperoxidase and myeloperoxidase.

Accordingly, there is a need for an improved method for removing biofilms that form on the body or within the body, particularly behind the eardrum. The improved method should be effective and safe. The improved method should also be compatible with antibiotics and other treatments for bacterial infection.

### SUMMARY OF THE INVENTION

This invention is directed to compositions that have the activity of removing biofilm, particularly in the ear. Compositions according to the present invention are suitable for treatment of ear infections such as otitis externa and otitis media, particularly those caused by *Pseudomonas aeruginosa.* The invention is directed to a composition for removal of biofilm in the ear comprising:
(a) a quantity of at least one peroxidase sufficient to exert a bactericidal action;
(b) an oxidase in a bactericidally effective quantity;
(c) at least one biofilm-dissolving enzyme selected from the group consisting of xylanase, β-glucanase, cellulase, α-galactosidase, glucanases, amylase, hyaluronidase, polygalacturonase (pectinase), dextranase, cellobiohydrolase, pullulanase, glycosylceramidase, glucan 1,4-a-glucosidase, oligo-1,6-glucosidase, fucoidanase, glycosylceramidase, glycosylceramidase, thioglucosidase, β-D-glucosidase and glycopeptide N-glycosidase;
(d) a pharmaceutically acceptable carrier suitable for administration into the ear canal;
(e) a quantity of lysozyme and lactoferrin sufficient to exert a bactericidal action;
(f) hydrocortisone to prevent or inhibit inflammation in the ear; and
(g) at least one substrate that can be converted to an ion with bactericidal properties by the enzymatic action of the peroxidase in a quantity such that an effective concentration of the ion with bactericidal properties is produced by the catalytic action of the peroxidase, selected from an alkali metal salt of thiocyanate, iodate, or chlorate,
and to its use for treating ear infection

One aspect of the present disclosure is a composition for removal of biofilm in the ear comprising:
(1) a quantity of at least one biofilm-dissolving enzyme that catalyzes the hydrolysis of a bond that connects two monosaccharides in a polysaccharide or that connects a monosaccharide with a protein molecule in a glycoprotein sufficient to break down biofilm in the ear; and
(2) a pharmaceutically acceptable carrier suitable for administration into the ear canal.

The at least one biofilm-dissolving enzyme that catalyzes the hydrolysis of a bond that connects two monosaccharides in a polysaccharide or that connects a monosaccharide with a protein molecule in a glycoprotein is selected from the group consisting of xylanase, β-glucanase, cellulase, α-galactosidase, glucanases, amylase, hyaluronidase, polygalacturonase (pectinase), dextranase, cellobiohydrolase, pullulanase, glycosylceramidase, glucan 1,4-α-glucosidase, oligo-1,6-glucosidase, fucoidanase, glycosylceramidase, glycosylceramidase, thioglucosidase, and glycopeptide N-glycosidase. Typically, the enzyme is selected from the group consisting of xylanase, β-glucanase, cellulase, α-galactosidase, glucanases, amylase, hyaluronidase, polygalacturonase (pectinase), dextranase, and cellobiohydrolase.

The composition comprises at least one ingredient in a quantity effective to prevent or inhibit inflammation in the ear. This is hydrocortisone.

The composition can further comprise an antibiotic that is effective in the treatment of *P. aeruginosa* in a quantity effective to exert a bactericidal action against *P*. *aeruginosa.*

The composition further includes lysozyme and lactoferrin. Additionally, the composition further includes at least one peroxidase in a quantity sufficient to exert a bactericidal action. A suitable peroxidase is lactoperoxidase. The composition further includes at least one substrate that can be converted to an ion with bactericidal properties by the enzymatic action of the peroxidase in a quantity such that an effective concentration of the ion with bactericidal properties is produced by the catalytic action of the peroxidase selected from an alkali metal salt of thiocyanate, iodate, or chlorate. The composition further includes an oxidase in a bactericidally effective quantity, such as glucose oxidase, as well as a substrate for the oxidase, such as glucose when the oxidase is glucose oxidase.

### DETAILED DESCRIPTION OF THE INVENTION

Antibiotics are the primary treatment for ear infection but, as discussed earlier, only kill free-floating bacteria. It is difficult if not impossible, for antibiotics to kill bacteria embedded in a biofilm.

By the application of a biofilm-dissolving enzyme system first to the ear or together with an antibiotic, the antibiotic is made much more effective.

In general, a biofilm-dissolving enzyme suitable for use in methods according to the present disclosure is an enzyme that catalyzes the hydrolysis of a bond that connects two monosaccharides in a polysaccharide or that connects a monosaccharide with a protein molecule in a glycoprotein. These enzymes are referred to herein as "glycoside linkage-hydrolyzing enzymes."

Biofilm-dissolving enzymes suitable for use in compositions according to the present disclosure include, but are not limited to, xylanase, β-glucanase, cellulase, α-galactosidase, glucanases, amylase, hyaluronidase, polygalacturonase (pectinase), dextranase, and cellobiohydrolase. Other hydrolytic enzymes that are capable of dissolving a bond that connects two monosaccharides in a polysaccharide or that connects a monosaccharide with a protein molecule in a glycoprotein can also be used, including, but not limited to, pullulanase, glycosylceramidase, glucan 1,4-α-glucosidase, oligo-1,6-glucosidase, fucoidanase, glycosylceramidase, glycosylceramidase, thioglucosidase, and glycopeptide N-glycosidase, as well as other enzymes.

Xylanase (EC 3.2.1.8), more precisely, endo-1,4-β-xylanase, is the name given to a class of enzymes that degrade the linear polysaccharide β-1,4-xylan into the monosaccharide xylose. Xylanase catalyzes the endohydrolysis of 1,4-β-D-xylosidic linkages in xylans. Xylanase is produced by many microorganisms, including *Thermomyces lanuginosus.* Information on xylanase is available at http://www.brenda.uni-koeln.de/php/result flat.php4?ecno=3.2.1.8.

β-glucanase (EC 3.2.1.6), more precisely, endo-1,3(4)- β-glucanase, is an enzyme that catalyzes the endohydrolysis of 1,3- or 1,4-linkages in β-D-glucans when the D-glucose residue whose reducing group is involved in the linkage to be hydrolyzed is itself substituted at C-3. Many sources of β-glucanase are known, particularly from plants and fungi, such as *Candida utilis* and *Saccharomyces cerevisiae.* Information on β-glucanase is available at http://www.brenda.uni-koeln.de/php/result flat.php4?ecno=3.2.1.6.

Cellulase (EC 3.2.1.4) is an enzyme that catalyzes the endohydrolysis of 1,4-β-D-glucosidic linkages in cellulose, lichenin and cereal β-D-glucans. Sources for cellulase include *Aspergillus niger, Clostridium thermocellum,* and *Cellulomonas fimi.* Information on cellulase is available at http://www.brenda.uni-koeln.de/php/result flat.php4?ecno=3.2.1.4.

α-galactosidase (EC 3.2.1.22) is an enzyme that catalyzes the hydrolysis of terminal, non-reducing alpha-D-galactose residues in α-D-galactosides, including galactose oligosaccharides, galactomannans and galactohydrolase. Sources of α-galactosidase include A. *niger, E. coli, Glycine max* (soybean), and *Lactobacillus plantarum.* Information on α-galactosidase is available at http://www.brenda.uni-koeln.de/php/result flat.php4?ecno=3.2.1.22.

Glucanase, or 1,3-β-D-glucosidase (EC 3.2.1.39), is an enzyme that catalyzes the hydrolysis of 1,3-β-D-glucosidic linkages in 1,3-β-D-glucans. Sources of glucanase include *Arabidopsis thaliana, C. thermocellum, Hordeum vulgare,* and *Oryza sativa.* Information on glucanase is available at http://www.brenda.uni-koeln.de/php/result flat.php4?ecno=3.2.1.39.

Amylase, more precisely α-amylase (EC 3.2.1.1) or β-amylase (EC 3.2.1.2), is a class of enzymes that hydrolyzes amylose, a component of starch. The enzyme α-amylase catalyzes the endohydrolysis of 1,4-α-D-glucosidic linkages in polysaccharides containing three or more 1,4-α-linked D-glucose units. Sources of α-amylase include *A. niger, Aspergillus oryzae, Bacillus licheniformis,* and *Bacillus stearothermophilus.* The enzyme β-amylase catalyzes the hydrolysis of 1,4-β-D-glucosidic linkages in polysaccharides so as to remove successive maltose units from the non-reducing ends of the chains. Sources of β-amylase include *H. vulgare* and *Bacillus cereus.* Information on α-amylase is available at http://www.brenda.uni-koeln.de/php/result flat.php4?ecno=3.2.1.1. Information on β-amylase is available at http://www.brenda.uni-koeln.de/php/result flat.php4?ecno=3.2.1.2.

Hyaluronidase, more precisely hyaluronate lyase (EC 4.2.2.1), catalyzes the cleavage of hyaluronate chains at a β-D-GalNAc-(1-4)-β-D-GlcA bond, ultimately breaking the polysaccharide down to 3-(4-deoxy-β-D-gluc-4-enuronosyl)-N-acetyl-D-glucosamine. Sources for hyaluronidase include *Candida albicans* and *Streptomyces griseus.* Information on hyaluronidase is available at http://www.brenda.uni-koeln.de/php/result flat.php4?ecno=4.2.2.1.

Polygalacturonase, also known as pectinase, and whose systematic name is poly(1,4-α-D-galacturonide) glycanohydrolase (EC 3.2.1.15), catalyzes the hydrolysis of 1,4-α-D-galactosiduronic linkages in pectate and other galacturonans. Sources for polygalacturonase include *A. niger* and *G*. *max.* Information on polygalacturonase is available at http://www.brenda.uni-koeln.de/php/result flat.php4?ecno=3.2.1.15. A suitable preparation of pectinase is marketed by Novo Nordisk as Pectinex Ultra SPL™.

Dextranase, whose systematic name is 1,6-α-D-glucan 6-glucanohydrolase, catalyzes the endohydrolysis of 1,6-α-D-glucosidic linkages in dextran. Sources of dextranase include *Penicillum funiculosum* and *Avena sativa.* Information on dextranase is available at http://www.brenda.uni-koeln.de/php/result flat.php4?ecno=3.2.1.11.

Cellobiohydrolase, also known as cellulase, and whose systematic name is 1,4-(1,3;1,4)-β-D-glucan 4-glucanohydrolase (EC 3.2.1.4), catalyzes the endohydrolysis of 1,4-β-D-glucosidic linkages in cellulose, lichenin and cereal β-D-glucans. Sources of cellobiohydrolase include *A*. *niger* and *Clostridium cellulolyticum.* Information on cellobiohydrolase is available at http://www.brenda.uni-koeln.de/php/result flat.php4?ecno=3.2.1.4.

One or more of these enzymes is included in a composition according to the present disclosure, together with a pharmaceutically acceptable carrier suitable for administration into the ear canal. Accordingly, one embodiment is a composition for removal of biofilm in the ear comprising:
(1) a quantity of at least one biofilm-dissolving enzyme that catalyzes the hydrolysis of a bond that connects two monosaccharides in a polysaccharide or that connects a monosaccharide with a protein molecule in a glycoprotein sufficient to break down biofilm in the ear; and
(2) a pharmaceutically acceptable carrier suitable for administration into the ear canal.

The pharmaceutically acceptable carrier suitable for administration into the ear canal can include buffers, ingredients to control the viscosity of the composition, preservatives, and other conventional ingredients as known in the art. Examples of specific ingredients included in the compositions are provided below in Formulation 1 and Formulation 2.

Typically, in compositions according to the present disclosure, the pharmaceutically acceptable carrier suitable for administration into the ear canal includes propylene glycol. Preferably, the pharmaceutically acceptable carrier suitable for administration into the ear canal includes propylene glycol and glycerol. In another preferred alternative, the pharmaceutically acceptable carrier suitable for administration into the ear canal includes propylene glycol, glycerol, and tripropylene glycol.

Compositions according to the present disclosure further includes at least one ingredient in a quantity effective to prevent or inhibit inflammation in the ear. A suitable ingredient is hydrocortisone.

Compositions according to the present disclosure can further include an antibiotic that is effective in the treatment of *P. aeruginosa* in a quantity effective to exert a bactericidal action against *P. aeruginosa.* The antibiotic included in a composition according to the present invention can be, for example, amikacin; a broad-spectrum penicillin such as, but not limited to, ticarcillin, piperacillin, mezlocillin, or azlocillin; ceftazidime; cefepime; ciprofloxacin; tobramycin; aztreonam; imipenem; or meropenem. Alternatively, an antibiotic such as the antibiotics recited above can be administered separately to promote killing of the bacteria in the biofilm. If administered separately, the antibiotic can be administered topically or systemically.

Compositions according to the present disclosure further includes a quantity of lysozyme, lactoferrin, and a peroxidase in a quantity sufficient to exert a bactericidal action. Typically, the peroxidase is selected from the group consisting of lactoperoxidase, myeloperoxidase, horseradish peroxidase, eosinophil peroxidase, and glutathione peroxidase. Preferably, the peroxidase is selected from the group consisting of lactoperoxidase and myeloperoxidase. More preferably, the peroxidase is lactoperoxidase. Lactoperoxidase is a glycoprotein which, in one commercial embodiment, is a lyophilized powder derived from milk. This commercial peroxidase has an activity of 80 IU/mg and a projected molecular weight of 93,000 for L-tyrosine iodination. The physicochemical properties reported for lactoperoxidase include a molecular weight of 78,000, a partial specific volume, reflective of the amino acid composition, of 0.74, and the presence of 1.0 mole of heme per mole of lactoperoxidase.

The composition further includes at least one substrate that can be converted to an ion with bactericidal properties by the enzymatic action of the peroxidase enzyme. The substrate is present in a quantity such that an effective concentration of the ion with bactericidal properties is produced by the catalytic action of the peroxidase enzyme. Suitable substrates include alkali metal salts of anions such as thiocyanate, iodate, or chlorate. The alkali metal salt is typically a sodium or potassium salt, although other alkali metal salts such as lithium or cesium can alternatively be used. The peroxidase enzyme catalyzes the conversion of thiocyanate into hypothiocyanite (⁻OSCN), molecular oxygen (O₂), and water. The peroxidase enzyme similarly catalyzes the conversion of iodate or chlorate to hypoiodite or hypochlorite. These anions possess bactericidal activity.

In an alternative composition according to the present disclosure that includes a peroxidase, a catalase inhibitor is further included. The effectiveness of the peroxidase enzyme can be affected by the presence of catalase, which is present in many tissues. Catalase competes with peroxidase for hydrogen peroxide. In order to reduce the loss of hydrogen peroxide through the presence of catalase, an effective amount of an enzymatic inhibitor that is specific for catalase can be advantageously incorporated into a composition according to the present invention. Suitable enzymatic inhibitors specific for catalase include, but are not limited to ascorbic salts such as sodium ascorbate, potassium ascorbate, calcium ascorbate, ascorbyl palmitate, or mixtures thereof, and can be included in a composition according to the invention. An effective concentration of ascorbic salt in compositions according to the present invention is from about 1 × 10⁻⁶ to about 1 x 10⁻⁴ millimole per gram of composition. Iron salts such as ferrous sulfate, ferrous chloride, or ferrous iodide can also be incorporated into a composition according to the present invention as a potentiator for the ascorbic salt in its role as catalase inhibitor. A particularly preferred iron salt is ferrous sulfate.

Compositions according to the present disclosure that include a peroxidase enzyme and the at least one substrate that can be converted to an ion with bactericidal properties by the enzymatic action of the peroxidase enzyme can also advantageously be formulated with an aminohexose in order to increase the yield or accumulation of oxidized anionic biocidal agent, the quantity of the aminohexose being effective to increase the yield or accumulation of oxidized anionic biocidal agent. Typically, the aminohexose is an aminoglucose, but other aminohexoses such as aminogalactose can alternatively be used. Typically, the aminoglucose is selected from the group consisting of glucosamine, N-acetylglucosamine, and mixtures thereof. The aminoglucose is typically present in the composition in a concentration of from about 0.0001 millimole to about 0.002 millimole per gram of composition. Preferably, the aminoglucose is present in the composition in a concentration of from about 0.0003 millimole to about 0.001 millimole per gram of composition.

Compositions according to the present disclosure that include a peroxidase further includes an oxidase in a bactericidally effective quantity and, optionally, a substrate for the oxidase in a bactericidally effective quantity. The oxidase oxidizes the substrate and produces hydrogen peroxide, which is then used as a substrate by the peroxidase if present. The use of an oxidase is only required if a peroxidase is also present.

The oxidoreductase enzyme is typically selected from the group consisting of glucose oxidase, galactose oxidase, urate oxidase, choline oxidase, D-amino acid oxidase, D-glutamate oxidase, glycine oxidase, glycolic oxidase, L-sorbose oxidase, alcohol oxidase, and amine oxidase. Other enzymes can alternatively be used, such as nitroethane oxidase, D-aspartate oxidase, L-aminoacid oxidase, pyridoxamine phosphate oxidase, ethanolamine oxidase, pyruvateoxidase, oxalate oxidase, hexose oxidase, cholesterol oxidase, aryl alcohol-,oxidase, pyridoxine 4-oxidase, dehydroorotate oxidase, lathosterol oxidase, sarcosine oxidase, N-methylaminoacid oxidase, N⁶-methyllysine oxidase, 6-hydroxy-L-nicotine oxidase, 6-hydroxy-D-nicotine oxidase, 3-hydroxyanthranilate oxidase, aldehyde oxidase, and xanthine oxidase, as described in U.S. Patent No. 4,340,448 to Schiller et al..

For these enzymes, glucose oxidase catalyzes the reaction of β-D-glucose, water, and oxygen to produce hydrogen peroxide and gluconic acid. Galactose oxidase catalyzes the reaction of D-galactose and oxygen to produce hydrogen peroxide and D-galacto-hexodialdose. Urate oxidase catalyzes the reaction of uric acid, water, and oxygen to produce hydrogen peroxide, allantoin, and carbon dioxide. Choline oxidase catalyzes the reaction of choline and oxygen to produce hydrogen peroxide and betaine aldehyde. D-amino acid oxidase catalyzes the reaction of D-amino acids such as D-proline, D-methionine, D-isoleucine, D-alanine, D-valine, or D-phenylalanine with water and oxygen to produce hydrogen peroxide, ammonia, and the α-keto acid corresponding to the D-amino acid being oxidized. D-glutamate oxidase catalyzes the reaction of D-glutamic acid, water, and oxygen to produce hydrogen peroxide, ammonia, and 2-ketoglutarate. Glycine oxidase catalyzes the reaction of glycine, water, and oxygen to produce hydrogen peroxide, ammonia, and glyoxylic acid. Glycolic acid oxidase (also known as 2-hydroxyacid oxidase) catalyzes the reaction of glycolic acid and oxygen to produce 2-ketoacetic acid and hydrogen peroxide. L-sorbose oxidase catalyzes the reaction of L-sorbose and oxygen to produce 5-dehydro-D-fructose and hydrogen peroxide. Alcohol oxidase catalyzes the reaction of a lower primary alcohol or an unsaturated alcohol and oxygen to produce the corresponding aldehyde and hydrogen peroxide. Amine oxidase catalyzes the reaction of an amine, typically a primary amine, but also, in some cases, a secondary or tertiary amine , water, and oxygen to produce the corresponding aldehyde, ammonia, and hydrogen peroxide. In an illustrative reaction, glucose oxidase catalyzes the reaction of β-D-glucose, water, and oxygen during application to the outer ear to produce hydrogen peroxide and gluconic acid.

The properties of a number of preferred oxidases suitable for use in compositions according to the present disclosure are known. For example, glucose oxidase from *Aspergillus niger* has been determined to have a molecular weight of 150,000 (Pazur et al. (1965)). The enzyme is a glycoprotein containing two molecules of the redox coenzyme flavin adenine dinucleotide (FAD). The amino acid composition has been determined. The isoelectric point of the enzyme is 4.2. The optimum pH of the enzyme is 5.5 with a broad pH range of from 4 to 7. Inhibitors of the enzyme include monovalent silver ions and divalent mercury and copper ions.

Galactose oxidase from *Dactylium dendroides* has a molecular weight of 42,000. It is a metalloenzyme containing one gram-atom of copper per mole. The amino acid composition has been determined. The optimum pH of the enzyme is 7.

Urate oxidase (uricase) from hog liver or beef liver has a molecular weight of 100,000. It is a metalloenzyme containing one gram-atom of copper per mole. The isoelectric point of the enzyme is 6.3. The optimum pH of the enzyme is 9.

D-amino acid oxidase from hog kidney has a molecular weight of 90,000. The enzyme is a glycoprotein containing two molecules of flavin adenine dinucleotide. The optimum pH of the enzyme is 9.1. Certain heavy metals are inhibitors of the enzyme.

The oxidizable substrate is typically present in the composition at a concentration of from about 0.015 millimoles per milliliter of liquid to about 0.6 millimoles per gram of composition. Preferably, the oxidizable substrate is present in the composition at a concentration of from about 0.025 millimoles per gram of composition to about 0.1 millimole per gram of composition. The salt that acts as an oxygen acceptor is typically present in the composition at a concentration of from about 0.0001 millimole to about 0.01 millimole per gram of composition. The salt that acts as an oxygen acceptor is preferably present in the composition at a concentration of from about 0.001 millimole to about 0.006 millimole per gram of composition.

Typically, the oxidoreductase enzyme is present in the composition in a concentration of from about 0.5 IU to about 500 IU per gram of composition. Preferably, the oxidoreductase enzyme is present in the composition in a concentration of from about 10 IU to about 40 IU per gram of composition. Oxidoreductase enzymes are supplied in dry or liquid form with the label specifying the concentration in International Units on a per gram or per milliliter basis, as appropriate.

A particularly preferred oxidase is glucose oxidase. If glucose oxidase is included in a composition according to the present invention, a preferred substrate for the glucose oxidase, to be included in the composition, is β-D-glucose. If another oxidase enzyme is used, appropriate substrates are described above.

In particular, the following combinations of glycoside linkage-hydrolyzing enzymes and peroxidases, if present, can be used in compositions according to the present disclosure: (1) pectinase as the glycoside linkage-hydrolyzing enzyme; (2) dextranase and pectinase as the glycoside linkage-hydrolyzing enzymes; (3) dextranase and pectinase as the glycoside linkage-hydrolyzing enzymes, plus lactoperoxidase as the peroxidase; (4) pectinase as the glycoside linkage-hydrolyzing enzyme, plus lactoperoxidase as the peroxidase; (5) dextranase and xylanase as the glycoside linkage-hydrolyzing enzymes; (6) α-galactosidase and amylase as the glycoside linkage-hydrolyzing enzymes; (7) pectinase and amylase as the glycoside linkage-hydrolyzing enzymes, plus lactoperoxidase as the peroxidase; (8) dextranase, pectinase, and β-D-glucosidase as the glycoside linkage-hydrolyzing enzymes, plus lactoperoxidase as the peroxidase; (9) dextranase, pectinase, and cellulase as the glycoside linkage-hydrolyzing enzymes, plus lactoperoxidase as the peroxidase; and (10) dextranase, pectinase, cellulase, amylase, and xylanase as the glycoside linkage-hydrolyzing enzymes, plus lactoperoxidase as the peroxidase. Other combinations are possible. These combinations can be combined with lysozyme and/or lactoferrin. Additionally, as indicated above, glucose oxidase or another oxidase is included as a source of peroxide, plus a substrate for the oxidase such as β-D-glucose.

Other ingredients generally known in the pharmaceutical art can be incorporated into compositions according to the present disclosure, including colorants, chelating agents, preservatives, and stabilizers, with the proviso that these additional ingredients do not inhibit the hydrolytic and oxidation-reduction reactions on which the activity of the compositions according to the present invention depend.

The composition can further comprise a thickener to provide the composition with an enzyme immobilizing viscosity which inhibits enzymatic action during processing and in packing. A preferred thickener is hydroxypropylcellulose (Klucel). Other thickeners are known in the art and can be alternatively used. These thickeners include hydroxymethyl cellulose, methyl cellulose, polyvinylpyrrolidone (PVP), PVM, PVM/MA copolymers, xanthan gum, and mixtures thereof.

The composition can be aqueous or non-aqueous. If the composition is aqueous, the concentration of water (w/w) typically is from about 0.150% to about 5.441%. However, the composition can be a non-aqueous composition with substantially no water content.

In one alternative, the composition includes from about 35% to about 75% of a hydrocarbon. Typically the hydrocarbon is an isoprenoid or a derivative of an isoprenoid. If the hydrocarbon is an isoprenoid or a derivative of an isoprenoid, preferably the hydrocarbon has from four to six isoprene units. More preferably, the hydrocarbon has six isoprene units. A particularly suitable hydrocarbon is squalene. Other hydrocarbons can be used.

In one preferred alternative, the composition is formulated to treat otitis media. As used herein, the terms "treat," "treating," "treatment," and analogous terminology does not imply a cure for otitis media or any other disease or condition; rather, this terminology is used to refer to any clinically detectable improvement in the disease or condition being treated, including, but not limited to, reduction in bacterial numbers or viability, reduction in fever, reduction in pain, reduction in hearing loss, reduction in fluid effusion, improvement in subjective well-being experienced by the patient, or any other clinically detectable improvement.

In another preferred alternative, the composition is formulated to treat otitis externa.

In another preferred alternative, the composition is formulated to treat infection by *Pseudomonas aeruginosa.*

The physical form of a composition according to the present invention can be, for example, a solution, a gel, a cream, or a solid, depending on the exact composition and the method of administration chosen, as well as the site of administration and whether the composition is intended to treat otitis media or otitis externa. If the solution is a gel, the viscosity of the gel can be chosen to provide efficient application by the user according to general principles of gel composition for pharmaceutical compositions. The particular gel former or gel formers used in a particular composition and their concentrations can be determined by one of ordinary skill in the art.

Compositions according to the present disclosure can include additional components, such as, but not limited to, a gel forming component, a lipophilic component, a wax, a skin soothing component, an emulsifier component, a bulk adding component, a gum component, or other components such as are generally used in pharmaceutical compositions intended for application to the ear canal, such as stabilizers, buffers, a colorant, a fragrance, or a preservative. In particular, compositions according to the present invention can include one or more of the following components: (1) benzyl alcohol; (2) glycerol; (3) dipropylene glycol; (4) tripropylene glycol; (5) xanthan gum; (6) PEG-20 almond glyceride; (7) an isopropyl ester of a long chain fatty acid selected from the group consisting of isopropyl myristate, isopropyl laurate, and isopropyl stearate, preferably isopropyl myristate; (8) aloe vera; (9) sodium polyacrylate/polyacrylic acid; (10) beeswax; (11) PEG-40 stearate; (12) polyethylene glycol; and (13) Polawax.

Compositions according to the present disclosure can be formulated by techniques known in the art, including techniques that are conventional in the cosmetic art and in the art of over-the-counter and prescription drug composition for blending lipid-soluble components and water-soluble components for the preparation of liquids, gels, creams, or suppositories. These mixing techniques include both manual and mechanical mixing, and include homogenization mixing and sweep mixing. The mixing techniques to be used can be chosen by one of ordinary skill in the art based on variables such as the viscosity of the components to be mixed and the volume of those components, as well as the relative proportion of lipid-soluble and water-soluble ingredients, the proportion of water, and the final physical form of the desired composition.

Particular embodiments of compositions according to the present disclosure include, but are not limited to the following:

Formulation 1 is an aqueous composition including the enzyme pectinase. In these formulations, percentages are given in terms of (w/w).

Formulation 1 is an aqueous composition containing a minimal amount of water. Formulation 1 includes lactoperoxidase, glucose oxidase, and pectinase, and β-D-glucose. Formulation 1 includes glycerol and propylene glycol, as well as hydroxypropylcellulose. Formulation 1 further includes hydrocortisone and benzyl alcohol, as well as lactoferrin and lysozyme.

Typically, Formulation 1 comprises:
(1) from about 28.328% to about 42.492% of glycerol;
(2) from about 52.761 % to about 64.485% of propylene glycol;
(3) from about 1.152% to about 1.728% of hydroxypropylcellulose;
(4) from about 2.405% to about 3.607% of benzyl alcohol;
(5) from about 0.120% to about 0.180% of water;
(6) from about 0.800% to about 1.200% of hydrocortisone;
(7) from about 0.241 % to about 0.361 % of β-D-glucose;
(8) from about 0.0064% to about 0.0096% of lactoperoxidase;
(9) from about 0.0008% to about 0.0012% of glucose oxidase;
(10) from about 0.0064% to about 0.0096% of lactoferrin;
(11) from about 0.0064% to about 0.0096% of lysozyme;
(12) from about 0.0080% to about 0.0120% of pectinase; and
(13) from about 0.028% to about 0.042% of potassium iodate.

Preferably, Formulation 1 comprises:
(1) about 35.410% of glycerol;
(2) about 58.623% of propylene glycol;
(3) about 1.440% of hydroxypropylcellulose;
(4) about 3.006% of benzyl alcohol;
(5) about 0.150% of water;
(6) about 1.000% of hydrocortisone;
(7) about 0.301 % of β-D-glucose;
(8) about 0.008% of lactoperoxidase;
(9) about 0.001 % of glucose oxidase;
(10) about 0.008% of lactoferrin;
(11) about 0.008% of lysozyme;
(12) about 0.010% of pectinase; and
(13) about 0.035% of potassium iodate.

Formulation 2 is the same as Formulation 1 except that it substitutes potassium thiocyanate in Formulation 2, for potassium iodate in Formulation 1.

Typically, Formulation 2 comprises:
(1) from about 28.328% to about 42.492% of glycerol;
(2) from about 52.761 % to about 64.485% of propylene glycol;
(3) from about 1.152% to about 1.728% of hydroxypropylcellulose;
(4) from about 2.405% to about 3.607% of benzyl alcohol;
(5) from about 0.120% to about 0.180% of water;
(6) from about 0.800% to about 1.200% of hydrocortisone;
(7) from about 0.241 % to about 0.361% of β-D-glucose;
(8) from about 0.0064% to about 0.0096% of lactoperoxidase;
(9) from about 0.0008% to about 0.0012% of glucose oxidase;
(10) from about 0.0064% to about 0.0096% of lactoferrin;
(11) from about 0.0064% to about 0.0096% of lysozyme;
(12) from about 0.0080% to about 0.0120% of pectinase; and
(13) from about 0.028% to about 0.042% of potassium thiocyanate.

Preferably, Formulation 2 comprises:
(1) about 35.410% of glycerol;
(2) about 58.623% of propylene glycol;
(3) about 1.440% of hydroxypropylcellulose;
(4) about 3.006% of benzyl alcohol;
(5) about 0.150% of water;
(6) about 1.000% of hydrocortisone;
(7) about 0.301 % of β-D-glucose;
(8) about 0.008% of lactoperoxidase;
(9) about 0.001 % of glucose oxidase;
(10) about 0.008% of lactoferrin;
(11) about 0.008% of lysozyme;
(12) about 0.010% of pectinase; and
(13) about 0.035% of potassium thiocyanate.

In another alternative, a composition according to the present disclosure can further include an antibiotic that is effective in the treatment of *P*. *aeruginosa* in a quantity effective to exert a bactericidal action against *P. aeruginosa.* These antibiotics are described above.

Other formulations can be prepared that are similar to the ones described in detail above.

Another embodiment of the present disclosure is a method of treating an ear infection comprising the step of administering a quantity of a composition according to the present invention as described above to a subject with an ear infection in order to treat the infection. The precise therapeutically effective amount for a subject will depend upon the subject's age, size, weight, and health, the extent of the ear infection, the bacterium causing the ear infection, the presence of other conditions such as allergic reactions that can complicate the ear infection, and the therapeutics or combination of therapeutics selected for administration, as well as variables such as liver and kidney function that affect the pharmacokinetics of administered therapeutics. Thus, it is not useful to specify an exact effective amount in advance. However, the effective amount for a given situation can be determined by routine experimentation and is within the judgment of the clinician. The frequency of administration, as well, can be determined by one of ordinary skill in the art with reference to the above parameters.

Typically, the ear infection is otitis externa or otitis media. Typically, the ear infection is caused by *Pseudomonas aeruginosa.*

The method of treating the ear infection can further comprise the administration of an antibiotic that is effective in the treatment of *P. aeruginosa* in a quantity effective to exert a bactericidal action against *P*. *aeruginosa,* the antibiotic being administered by a route other than route of administration of the composition according to the present invention. If the composition according to the present invention includes an antibiotic, the antibiotic that is administered by the route other than route of administration of the composition according to the present invention can be the same antibiotic included in the composition or can be a different antibiotic. If the composition according to the present invention does not include an antibiotic, the antibiotic administered by the additional route can be any of the antibiotics described above as being effective in the treatment of *P. aeruginosa.* The route of administration, dose administered, and the frequency of administration can be determined by one of ordinary skill in the art by reference to the parameters described above, such as the subject's age, size, weight, and health, the extent of the ear infection, the bacterium causing the ear infection, the presence of other conditions such as allergic reactions that can complicate the ear infection, and the therapeutics or combination of therapeutics selected for administration, as well as variables such as liver and kidney function that affect the pharmacokinetics of administered therapeutics, and the properties of the antibiotic such as its molecular weight and relative degree of hydrophobicity or hydrophilicity, as well as its susceptibility to hydrolysis in the digestive tract. Typically, administration of the antibiotic administered by the additional route is by the oral or parenteral route; if parenteral, typically the antibiotic is administered intramuscularly. In some cases of severe infection, intravenous administration can be required. Information on specific antibiotics and optimum routes of administration can be found, for example, in J.G. Hardman & L.E. Limbird, eds., "Goodman & Gilman's The Pharmacological Basis of Therapeutics" (10th ed., McGraw-Hill, New York, 2001).

### ADVANTAGES OF THE INVENTION

The present invention provides a safe and effective means for treating ear infections, particularly otitis externa and otitis media, and particularly those ear infections caused by *P*. *aeruginosa.* Compositions and methods according to the present invention, in removing bacteria-laden biofilm, not only provide for more effective treatment of such infections, but also prevent recurrence of the infections, such as otitis externa and otitis media. Compositions and methods according to the present disclosure are suitable for use with other treatment modalities, such as antibiotic administration, and do not cause inflammation or other side effects.

Accordingly, compositions and methods according to the present disclosure possess industrial applicability for the preparation of medicaments for the treatment of ear infections, especially otitis externa and otitis media, and especially for the treatment of infections caused by *P*. *aeruginosa.*

The inventions illustratively described herein can suitably be practiced in the absence of any element or elements, limitation or limitations, not specifically disclosed herein. Thus, for example, the terms "comprising," "including," "containing," etc. shall be read expansively and without limitation. Additionally, the terms and expressions employed herein have been used as terms of description and not of limitation, and there is no intention in the use of such terms and expressions of excluding any equivalents of the future shown and described or any portion thereof, and it is recognized that various modifications are possible within the scope of the invention claimed.

It is also to be understood that the above description is intended to be illustrative and not restrictive. Many embodiments will be apparent to those of ordinary skill in the art upon reviewing the above description. The scope of the invention should therefore, be determined not with reference to the above description, but should instead be determined with reference to the appended claims, along with the full scope of equivalents to which such claims are entitled.

## Claims

1. A composition for removal of biofilm in the ear comprising:
(a) a quantity of at least one peroxidase sufficient to exert a bactericidal action;
(b) an oxidase in a bactericidally effective quantity;
(c) at least one biofilm-dissolving enzyme selected from the group consisting of xylanase, β-glucanase, cellulase, α-galactosidase, glucanases, amylase, hyaluronidase, polygalacturonase (pectinase), dextranase, cellobiohydrolase, pullulanase, glycosylceramidase, glucan 1,4-a-glucosidase, oligo-1,6-glucosidase, fucoidanase, glycosylceramidase, glycosylceramidase, thioglucosidase, β-D-glucosidase and glycopeptide N-glycosidase;
(d) a pharmaceutically acceptable carrier suitable for administration into the ear canal;
(e) a quantity of lysozyme and lactoferrin sufficient to exert a bactericidal action;
(f) hydrocortisone to prevent or inhibit inflammation in the ear; and
(g) at least one substrate that can be converted to an ion with bactericidal properties by the enzymatic action of the peroxidase in a quantity such that an effective concentration of the ion with bactericidal properties is produced by the catalytic action of the peroxidase, selected from an alkali metal salt of thiocyanate, iodate, or chlorate.

2. The composition according to claim 1 wherein the pharmaceutically acceptable carrier suitable for administration into the ear canal is selected from the group consisting of propylene glycol, glycerol and tripropylene glycol.

3. The composition according to claim 1 wherein the composition further comprises a substance in a quantity sufficient to exert a bactericidal action against *Pseudomonas aeruginosa* selected from the group consisting of amikacin, ticarcillin, piperacillin, mezlocillin, azlocillin, ceftazidime, cefepime, ciprofloxacin, tobramycin, aztreonam, imipenem, and meropenem.

4. The composition according to claim 1 wherein the at least one peroxidase is selected from the group consisting of lactoperoxidase, myeloperoxidase, horseradish peroxidase, eosinophil peroxidase, and glutathione peroxidase.

5. The composition according to claim 1 wherein the composition further includes a catalase inhibitor.

6. The composition according to claim 1 wherein the composition further includes an aminohexose in a quantity effective to increase the yield or accumulation of oxidized anionic biocidal agent.

7. The composition according to claim 1 wherein the oxidase is selected from the group consisting of glucose oxidase, galactose oxidase, urate oxidase, choline oxidase, D-amino acid oxidase, D-glutamate oxidase, glycine oxidase, glycolic oxidase, L-sorbose oxidase, alcohol oxidase, and amine oxidase.

8. The composition according to claim 1 wherein the composition further includes squalene.

9. The composition according to any preceding claim for use in the treatment of an ear infection.

## Patentansprüche

1. Zusammensetzung zur Entfernung eines Biofilms im Ohr umfassend:
(a) eine Menge von mindestens einer Peroxidase, die ausreichend ist, eine bakterizide Wirkung auszuüben;
(b) eine Oxidase in einer bakterizid wirksamen Menge;
(c) mindestens ein Biofilm-auflösendes Enzym ausgewählt aus der Gruppe bestehend aus Xylanase, β-Glucanase, Cellulase, α-Galactosidase, Glucanasen, Amylase, Hyaluronidase, Polygalacturonase (Pectinase), Dextranase, Cellobiohydrolase, Pullulanase, Glycosylceramidase, Glucan-1,4-α-glucosidase, Oligo-1,6-glucosidase, Fucoidanase, Glycosylceramidase, Glycosylceramidase, Thioglucosidase, β-D-Glucosidase und Glycopeptid-N-glycosidase;
(d) einen pharmazeutisch verträglichen Träger, der für eine Verabreichung in den Gehörgang geeignet ist;
(e) eine Menge an Lysozym und Lactoferrin, die ausreichend ist, eine bakterizide Wirkung auszuüben;
(f) Hydrocortison zum Verhindern oder Inhibieren einer Entzündung im Ohr; und
(g) mindestens ein Substrat, das durch die enzymatische Wirkung der Peroxidase zu einem Ion mit bakteriziden Eigenschaften umgewandelt werden kann, in einer derartigen Menge, dass durch die katalytische Wirkung der Peroxidase eine wirksame Konzentration des Ions mit bakteriziden Eigenschaften erzeugt wird, ausgewählt aus einem Alkalimetallsalz von Thiocyanat, Iodat oder Chlorat.

2. Zusammensetzung nach Anspruch 1, wobei der pharmazeutisch verträgliche Träger, der für eine Verabreichung in den Gehörgang geeignet ist, aus der Gruppe ausgewählt ist bestehend aus Propylenglycol, Glycerol und Tripropylenglycol.

3. Zusammensetzung nach Anspruch 1, wobei die Zusammensetzung ferner eine Substanz in einer Menge umfasst, die ausreichend ist, gegenüber *Pseudomonas aeruginosa* eine bakterizide Wirkung auszuüben, ausgewählt aus der Gruppe bestehend aus Amikacin, Ticarcillin, Piperacillin, Mezlocillin, Azlocillin, Ceftazidim, Cefepim, Ciprofloxacin, Tobramycin, Aztreonam, Imipenem und Meropenem.

4. Zusammensetzung nach Anspruch 1, wobei die mindestens eine Peroxidase aus der Gruppe ausgewählt ist bestehend aus Lactoperoxidase, Myeloperoxidase, Meerrettichperoxidase, Eosinophilperoxidase und Glutathionperoxidase.

5. Zusammensetzung nach Anspruch 1, wobei die Zusammensetzung ferner einen Katalaseinhibitor umfasst.

6. Zusammensetzung nach Anspruch 1, wobei die Zusammensetzung ferner eine Aminohexose in einer Menge umfasst, die wirksam ist für eine Erhöhung der Ausbeute oder Akkumulation eines oxidierten anionischen bioziden Mittels.

7. Zusammensetzung nach Anspruch 1, wobei die Oxidase aus der Gruppe ausgewählt ist bestehend aus Glucoseoxidase, Galactoseoxidase, Uratoxidase, Cholinoxidase, D-Aminosäureoxidase, D-Glutamatoxidase, Glycinoxidase, Glycoloxidase, L-Sorboseoxidase, Alkoholoxidase und Aminoxidase.

8. Zusammensetzung nach Anspruch 1, wobei die Zusammensetzung ferner Squalen umfasst.

9. Zusammensetzung nach einem der vorhergehenden Ansprüche zur Verwendung bei der Behandlung einer Ohrinfektion.

## Revendications

1. Composition pour l'élimination d'un biofilm dans l'oreille comprenant :
(a) une quantité d'au moins une peroxydase suffisante pour exercer une action bactéricide ;
(b) une oxydase en une quantité bactéricidement efficace ;
(c) au moins une enzyme dissolvant les biofilms choisie dans le groupe constitué par la xylanase, la β-glucanase, la cellulase, l'α-galactosidase, les glucanases, l'amylase, l'hyaluronidase, la polygalacturonase (pectinase), la dextranase, la cellobiohydrolase, la pullulanase, la glycosylcéramidase, la glucane 1,4-α-glucosidase, l'oligo-1,6-glucosidase, la fucoïdanase, la glycosylcéramidase, la glycosylcéramidase, la thioglucosidase, la β-D-glucosidase et le glycopeptide N-glycosidase ;
(d) un véhicule pharmaceutiquement acceptable convenant à l'administration dans le conduit auditif ;
(e) une quantité de lysozyme et de lactoferrine suffisante pour exercer une action bactéricide ;
(f) de l'hydrocortisone pour prévenir ou inhiber l'inflammation dans l'oreille ; et
(g) au moins un substrat qui peut être converti en ion ayant des propriétés bactéricides par l'action enzymatique de la peroxydase en une quantité telle qu'une concentration efficace de l'ion ayant des propriétés bactéricides est produite par l'action catalytique de la peroxydase, choisi parmi un sel de métal alcalin de thiocyanate, iodate, ou chlorate.

2. Composition selon la revendication 1 dans laquelle le véhicule pharmaceutiquement acceptable convenant à l'administration dans le conduit auditif est choisi dans le groupe constitué par le propylène glycol, le glycérol et le tripropylène glycol.

3. Composition selon la revendication 1 dans laquelle la composition comprend en outre une substance en une quantité suffisante pour exercer une action bactéricide contre *Pseudomonas aeruginosa* choisie dans le groupe constitué par l'amikacine, la ticarcilline, la pipéracilline, la mezlocilline, l'azlocilline, le ceftazidime, le céfépime, la ciprofloxacine, la tobramycine, l'aztréonam, l'imipénem, et le méropenem.

4. Composition selon la revendication 1 dans laquelle ladite au moins peroxydase est choisie dans le groupe constitué par la lactoperoxydase, la myéloperoxydase, la peroxydase du raifort, la peroxydase éosinophile, et la glutathione peroxydase.

5. Composition selon la revendication 1 dans laquelle la composition contient en outre un inhibiteur de catalase.

6. Composition selon la revendication 1 dans laquelle la composition contient en outre un aminohexose en une quantité efficace pour accroître le rendement ou l'accumulation de l'agent biocide anionique oxydé.

7. Composition selon la revendication 1 dans laquelle l'oxydase est choisie dans le groupe constitué par la glucose oxydase, la galactose oxydase, l'urate oxydase, la choline oxydase, l'acide D-aminé oxydase, la D-glutamate oxydase, la glycine oxydase, l'oxydase glycolique, la L-sorbose oxydase, l'alcool oxydase, et l'amine oxydase.

8. Composition selon la revendication 1 dans laquelle la composition contient en outre un squalène.

9. Composition selon l'une quelconque des revendications précédentes destinée à être utilisée dans le traitement d'une infection de l'oreille.
